# EUROPEAN PATENT APPLICATION

(11) **EP 1 662 260 A1**
(43) Date of publication of application: **31.05.2006**
(21) Application number: 04772437.2
(22) Date of filing: 30.08.2004
(51) Int. Cl.: G01N 33/68

(54) **METHOD OF ANALYZING PROTEIN AND PROTEIN ANALYSIS REAGENT TO BE USED THEREIN**

(30) Priority: 03.09.2003 JP 2003311624
(71) Applicant: Arkray, Inc., Kyoto-shi, Kyoto 601-8045 (JP)
(72) Inventor: NISHINO, Susumu, c/o Arkray, Inc., Kyoto-shi, Kyoto 601-8045 (JP); OKAMOTO, Masashi, c/o Arkray, Inc., Kyoto-shi, Kyoto 601-8045 (JP)
(74) Representative: Goddard, Christopher Robert
(86) International application number: PCT/JP2004/012481
(87) International publication number: WO 2005/024430

(57) **Abstract**

The present invention provides a method for analyzing protein using a Biuret reaction, which can prevent coagulation of protein and an increase of a viscosity of a reaction liquid caused thereby, even when using a sample including protein with a high concentration. A sample including protein is adjusted to be alkaline by adding a sodium hydroxide solution or the like, to which a divalent copper salt and a salt containing a chaotropic ion are added so as to cause a Biuret reaction, and a degree of coloring thereof is measured, thereby analyzing the protein. The chaotropic ion may be, for example, a SCN ion, an I ion, a NO₃ ion, a ClO₄ ion, a Br ion or a Cl ion.

## Description

### Technical Field

The present invention relates to a method for analyzing protein, and an analytical reagent for protein used therefor.

### Background Art

Protein in samples is analyzed in various fields such as medical, pharmaceutical, biological and agricultural fields. A method for analyzing protein is, for example, a dry weight method, a Kjeldahl method, a Biuret method, a Lowry method, a bicinchoninic acid method, an ultraviolet absorption method, a dye-coupling method, a turbidimetric method or the like. Among them, the Biuret method is commonly used, because the operation thereof is simple, coloring rates per unit weight have little differences even in the case where kinds of protein in samples are different, etc.

The Biuret method is a method for analyzing protein by utilizing a mechanism where a complex is formed by a reaction of protein and copper ions so as to be colored in purple. By measuring this color reaction optically as absorbance or a reflection rate, a concentration of protein can be measured (see, for example, Non-patent document 1). As a source of the copper ions, copper sulfate is used.
Non-patent document 1: "New biochemical experiment course, Protein I, separation/refinement/property", The Japanese Biochemical Society, TOKYO KAGAKU DOZIN CO., LTD., 1990, page 91

### Disclosure of Invention

However, the Biuret method has a problem where a viscosity of a reaction liquid is increased due to coagulation of protein. This increase of viscosity is a serious problem for a protein sample with a high concentration. For example, a sample analyzing tool (hereinafter, also called a test piece) used in a clinical examination is obtained by supporting a Biuret reagent in a dry state in a carrier such as a filter paper, to which a biological sample such as urine and blood is supplied directly so as to react with the Biuret reagent. Therefore, a concentration of protein in the reaction liquid is high. Accordingly, if the coloring by the Biuret reaction is analyzed optically in the state of the high viscosity, a problem occurs in analytical precision.

In the light of the above-described problems, an object of the present invention is to provide a method for analyzing protein with excellent analytical precision.

In order to attain the above-mentioned object, the method for analyzing protein of the present invention is a method for analyzing protein in a sample, in which the sample is alkaline, wherein a divalent copper salt and a salt containing a chaotropic ion are added to the sample so as to cause a Biuret reaction, and the Biuret reaction is analyzed optically.

The method for analyzing protein of the present invention can prevent coagulation of protein and an increase of a viscosity caused thereby, thus having excellent analytical precision. According to the present invention, in particular, when protein with a high concentration is used, the increase of the viscosity of the reaction liquid can be prevented, whereby the concentration of the protein can be analyzed with high precision.

### Brief Description of Drawings

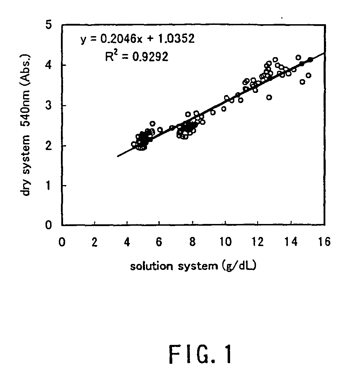

FIG. 1 is a graph showing a correlation between Example 1 and Comparative Example 1.

### Description of the Invention

In order to attain the above-mentioned object, the inventors of the present invention have made a series of studies mainly for finding the cause of the coagulation of protein. In the process of the studies, they found that copper sulfate used conventionally in the Biuret method causes the coagulation of protein. Moreover, in further studies, they found that use of a salt containing a chaotropic ion can prevent the coagulation of protein, thus leading to the present invention.

The reason why a chaotropic ion prevents the coagulation of protein is considered to be as mentioned below. Hydrophobic bonds have a large contribution to the coagulation of protein. Whereas, a chaotropic ion has an effect of increasing the water-solubility of hydrophobic molecules in water and degrade power of the hydrophobic bonds. Thus, it is considered that the coagulation of protein is prevented by the effect of the chaotropic ion to degrade the power of the hydrophobic bonds of the protein.

In the method for analyzing protein of the present invention, it is preferable to use a divalent copper salt of a chaotropic ion as the divalent copper salt and the salt containing the chaotropic ion.

In the analysis method of the present invention, the chaotropic ion may be, for example, a SCN ion, an I ion, a NO₃ ion, a ClO₄ ion, a Br ion, a Cl ion or the like. Among them, a SCN ion, an I ion, a NO₃ ion, a ClO₄ ion and a Br ion are preferable, and a SCN ion, a NO₃ ion and a ClO₄ ion are more preferable.

In the analysis method of the present invention, the divalent copper salt may be, for example, CuSO₄, Cu(ClO₄)₂, Cu(NO₃)₂, CuBr₂, CuCl₂, Cu(CH₃COO)₂ or the like. Among them, Cu(ClO₄)₂, Cu(NO₃)₂, CuBr₂ and CuCl₂ are preferable, and Cu(ClO₄)₂ is more preferable.

In the analysis method of the present invention, the divalent copper salt of the chaotropic ion may be, for example, Cu(NO₃)₂, Cu(ClO₄)₂, CuBr₂, CuCl₂ or the like. Among them, Cu(NO₃)₂, Cu(ClO₄)₂ and CuBr₂ are preferable, and Cu(ClO₄)₂ is more preferable.

In the analysis method of the present invention, in the case of performing a liquid system analysis or using a diluted sample, an amount of the chaotropic ion to be added ranges, for example, from 0.5 mM to 100 mM, and preferably ranges from 1 mM to 10 mM.

In the analysis method of the present invention, in the case of performing the liquid system analysis or using the diluted sample, an amount of the divalent copper salt to be added ranges, for example, from 0.5 mM to 100 mM, and preferably ranges from 1 mM to 10 mM.

In the analysis method of the present invention, in the case of performing the liquid system analysis or using the diluted sample, an amount of the divalent copper salt of the chaotropic ion to be added ranges, for example, from 0.5 mM to 100 mM, and preferably ranges from 1 mM to 10 mM.

In addition, a dilution concentration of the sample may be, for example, 1/50. Moreover, in the present invention, the sample may be used without dilution even in a liquid system analysis, and the diluted sample may be used even in a dry system analysis. The liquid system analysis is an analysis method using a reagent liquid, and is used in, for example, general experiments or the like. The dry system analysis is an analysis method using a reagent and the like in a dry state, and is applied to, for example, a sample analyzing tool (or a test piece) used in a clinical examination or the like.

In the analysis method of the present invention, in the case of performing the dry system analysis or using the sample without dilution, the amount of the chaotropic ion to be added ranges, for example, from 100 mM to 2000 mM, and preferably ranges from 500 mM to 1000 mM.

In the analysis method of the present invention, in the case of performing the dry system analysis or using the sample without dilution, the amount of the divalent copper salt to be added ranges, for example, from 100 mM to 1000 mM, and preferably ranges from 200 mM to 500 mM.

In the analysis method of the present invention, in the case of performing the dry system analysis or using the sample without dilution, the amount of the divalent copper salt of the chaotropic ion to be added ranges, for example, from 100 mM to 1000 mM, and preferably ranges from 200 mM to 500 mM.

In the present invention, another reagent such as an alkaline agent may be used besides the divalent copper salt and the salt containing the chaotropic ion, or the divalent copper salt of the chaotropic ion. The alkaline agent may be, for example, NaOH, KOH, LiOH, Ca(OH)₂ or the like. Either of them may be used preferably, but LiOH is particularly useful when being processed into the analyzing tool.

In the analysis method of the present invention, the sample may be alkaline when the chaotropic ion and the divalent copper ion react with the protein in the sample, that is, for example, the sample may be prepared to be alkaline in advance. Alternatively, the alkaline agents as described above may be added when the chaotropic ion and the divalent copper ion are mixed with the sample. In the analysis method of the present invention, a pH of the alkaline sample during the reaction is, for example, 9 or more, and preferably is 11 or more.

In the analysis method of the present invention, the concentration of the protein in the sample ranges, for example, from 0 g/dL (100 ml) to 15 g/dL (100 ml). This range is equivalent to a concentration range in which a measurement of protein is performed by an usual Biuret reaction.

In addition, in the present invention, the use of the term protein includes polypeptide.

In the analysis method of the present invention, the optical analysis may be, for example, an analysis of a degree of coloring of the sample caused by a Biuret reaction. Moreover, the analysis of the degree of the coloring of the sample may be performed by a visual evaluation, or by spectrophotometry or reflectometry using equipment. Furthermore, the optical analysis may be either a qualitative analysis or a quantitative analysis.

Next, the reagent of the present invention is an analytical reagent for protein used in the method for analyzing protein of the present invention, including a divalent copper salt and a salt containing a chaotropic ion.

In the reagent of the present invention, the chaotropic ion and the divalent copper salt may be the above-described chaotropic ion and the above-described divalent copper salt. Moreover, the reagent may include another reagent such as an alkaline agent besides the divalent copper salt and the salt containing the chaotropic ion. The another reagent such as the alkaline agent may be the above-described reagent.

Moreover, the reagent of the present invention is an analytical reagent for protein used in the method for analyzing protein of the present invention, including a divalent copper salt of a chaotropic ion.

In the reagent of the present invention, the divalent copper salt of the chaotropic ion may be, for example, the above-described divalent copper salt of the chaotropic ion. Moreover, the reagent may include another reagent such as an alkaline agent besides the divalent copper salt of the chaotropic ion. The another reagent such as the alkaline agent may be the above-described reagent.

The reagent of the present invention may be used by, for example, being dropped into the sample, being dried on a microplate by freeze drying, being disposed with a sample analyzing tool as mentioned below, or the like.

Next, the sample analyzing tool of the present invention is used in the method for analyzing protein of the present invention, and includes a carrier and a reagent, wherein the reagent includes a divalent copper salt and a salt containing a chaotropic ion.

In the sample analyzing tool of the present invention, the chaotropic ion and the divalent copper salt may be the above-described chaotropic ion and the above-described divalent copper salt. Moreover, in the sample analyzing tool of the present invention, the reagent may include another reagent such as an alkaline agent besides the divalent copper salt and the salt containing the chaotropic ion. The another reagent such as the alkaline agent may be the above-described reagent.

Moreover, the sample analyzing tool of the present invention is used in the method for analyzing protein of the present invention, and includes a carrier and a reagent, wherein the reagent includes a divalent copper salt of a chaotropic ion.

In the sample analyzing tool of the present invention, the divalent copper salt of the chaotropic ion may be the above-described divalent copper salt of the chaotropic ion. Moreover, in the sample analyzing tool of the present invention, the reagent may include another reagent such as an alkaline agent besides the divalent copper salt of the chaotropic ion. The another reagent such as the alkaline agent may be the above-described reagent.

Next, the method for analyzing protein of the present invention can be performed, for example, as described below.

An example where the analysis method of the present invention is applied to the liquid system analysis will be described. For example, a sample including protein, such as blood, is suspended in water as necessary, and an alkaline agent further is added so as to control a pH thereof to be in the above-described predetermined range. When a reagent that includes the divalent copper salt and the salt containing the chaotropic ion is added to the sample solution, which is subsequently stirred, the protein in the sample and a copper ion form a complex, and then coloring occurs, for example, after 10 seconds to 15 minutes from the addition of the reagent. This change may be observed visually, or measured by using an optical measurement apparatus such as a spectrophotometer. By measuring the degree of this coloring, a qualitative or quantitative analysis of the protein can be performed. If, for example, a calibration curve is prepared in advance, a concentration of the protein can be determined by the degree of the coloring caused by the Biuret reaction. A temperature of the reaction ranges, for example, from a room temperature to around 37°C, which is the usual adjusted temperature in a general analyzer.

Next, an example where the analysis method of the present invention is applied to the sample analyzing tool will be described. For example, the divalent copper salt of the chaotropic ion is dissolved in alkaline water so as to prepare a reagent solution. A pH of this reagent solution is controlled to be in a range that provides the predetermined pH when the sample is added. Then, a porous carrier such as a filter paper is impregnated with the reagent solution, and is dried thereafter. The carrier is not particularly limited, but for example, a porous sheet made of a plastic can be used as well as the filter paper. The carrier that supports the reagent as mentioned above may be used directly as the sample analyzing tool. Alternatively, this carrier that is further supported by another substrate sheet or the like may be used as the sample analyzing tool. When supplying a sample including protein, such as blood, to the carrier of such a sample analyzing tool, coloring occurs, for example, after 10 seconds to 15 minutes from the supply of the sample. The degree of this coloring is observed visually, or is measured by a reflectometer or the like so that a qualitative or quantitative analysis of the protein can be performed. If, for example, a calibration curve is prepared in advance, a concentration of the protein can be determined by the degree of the coloring caused by the Biuret reaction.

Also, for example, the carrier may be made of an optically transparent material and include a passage for the sample solution. In this case, a reagent including the divalent copper salt of the chaotropic ion may be disposed in the passage for the solution.

### Example 1

A reagent was prepared and an analysis of protein was performed as described below.

Human serum in which human serum albumin (manufactured by Sigma Chemical Co.) was added and human serum in which human serum albumin was not added were used for samples (120 examples).

The below-described microplate reagent was prepared, which was subsequently poured dividedly into 96 Well Microplate by 50 µL each, and was subjected to freeze drying.

| (Microplate reagent) | |
|---|---|
| tartaric acid | 9.0 g |
| lithium hydroxide | 12.6 g |
| copper perchlorate | 14.8 g |
| distilled water | 100.0 g |

Next, 50 µL of each of the samples was added in the freeze-dried reagent, which reacted at a room temperature for 8 minutes, and thereafter, absorbance thereof at a wavelength of 540 nm was measured. For measuring the absorbance, a microplate absorbance measurement apparatus (manufactured by Thermo Labsystems, trade name: Multiskan Ascent) was used.

### Comparative Example 1

5 µL of each sample that was the same as the sample of Example 1 was mixed with 350 µL of the below-described solution-system reagent, which reacted at a room temperature for 10 minutes, and thereafter, absorbance thereof at a wavelength of 546 nm was measured. For measuring the absorbance, an automatic analyzer (manufactured by Hitachi Ltd., trade name: Type 7070) was used.

| (Solution-system reagent) | |
|---|---|
| tartaric acid | 9.0 g |
| sodium hydroxide | 12.6 g |
| copper sulfate | 14.8 g |
| potassium iodide | 0.1 g |
| distilled water | 100.0 g |

Next, a calibration curve was obtained by using a bovine serum albumin solution with a known concentration and distilled water so as to calculate a concentration of the protein.

FIG. 1 is a graph showing a result of a correlation between Example 1 and Comparative Example 1, which was obtained from comparison therebetween, where the formula in the figure represents the correlation, and R² represents a correlation coefficient thereof. In addition, in FIG. 1, Example 1 represents a dry method and Comparative Example 1 represents a solution method.

As shown in FIG. 1, there was a high correlation between Example 1 and Comparative Example 1.

### Example 2

A reagent and a sample solution were prepared, and an analysis of protein was performed, as described below.

A human serum albumin (HSA) solution (manufactured by Sigma Chemical Co.) was prepared to have a concentration of 30 g/dL(100 mL), which subsequently was used to prepare HSA solutions with the below-described concentrations.

The below-described reagent solution A was prepared, and 200 µL of the reagent solution A and 400 µL of each sample reacted with each other at a room temperature for 8 minutes. Then, the thus obtained reaction liquid was poured dividedly into transparent cells with cell lengths of 1 mm, and the absorbance thereof at a wavelength of 550 nm was measured by using an absorptiometer (manufactured by JASCO Corporation, trade name: V-550). The result thereof will be shown below in Table 1.

| (Reagent solution A) | |
|---|---|
| tartaric acid | 1.35 g (2.000 M) |
| lithium hydroxide | 1.89 g (10.000 M) |
| copper perchlorate | 2.22 g (1.333 M) |
| distilled water | 5.00 g |

### Comparative Example 2

Absorbance was measured in the same manner as Example 2, except using the below-described reagent solution B as the reagent solution. The result thereof will be shown below in Table 1.

| (Reagent solution B) | |
|---|---|
| tartaric acid | 1.35 g (2.000 M) |
| lithium hydroxide | 1.89 g (10.000 M) |
| copper sulfate | 1.50 g (1.333 M) |
| distilled water | 5.00 g |

**(Table 1)**

| Concentration of HSA (g/dL) | | Example 2 | | Comparative Example 2 | |
|---|---|---|---|---|---|
| Dilution concentration | Final concentration | Viscosity | Absorbance | Viscosity | Absorbance |
| 0 | 0 | Y | 0.33 | Y | 0.32 |
| 3 | 2 | Y | 0.97 | Y | 0.97 |
| 6 | 4 | Y | 1.52 | Y | 1.52 |
| 9 | 6 | Y | 1.97 | Y | 2.01 |
| 12 | 8 | Y | 2.32 | Y | 2.40 |
| 15 | 10 | Y | 2.59 | Y | 2.68 |
| 16.5 | 11 | Y | 2.65 | N | - |
| 18 | 12 | Y | 2.77 | N | - |
| 19.5 | 13 | Y | 2.92 | N | - |

In Table 1 above, the letter Y represents the capability of the reaction liquid to be poured dividedly into the cells, the letter N represents the incapability of the reaction liquid to be poured dividedly into the cells due to its high viscosity, and the sign of - represents impossibility of measuring the absorbance due to the incapability of the reaction liquid to be poured dividedly into the cells. Here, the incapability of the reaction liquid to be poured dividedly means the state where the reaction liquid could not move into the cells, or the state where the reaction liquid could not be aspirated with a pipet.

As shown above in Table 1, in Comparative Example 2 (the conventional method), when the final concentration of HSA (protein) was 11 g/dL or more, the viscosity increased to a level that did not allow to be poured dividedly into the cells. On the other hand, as shown in Example 2, even the sample with a high final concentration of HSA (protein) that is more than 10 g/dL could be subjected to the measurement with high analytical precision. Thereby, according to the present invention, even in the sample analyzing tool in which a dry reagent was prepared, the dry reagent could be dissolved completely, so that the sample and the reagent could be mixed uniformly.

### Industrial Applicability

The method for analyzing protein of the present invention can prevent coagulation of protein and an increase of a viscosity caused thereby, thus having excellent analytical precision. Therefore, according to the analysis method of the present invention, precision of the analysis of protein in various fields such as medical, pharmaceutical and biochemical fields can be improved. In particular, the method for analyzing protein of the present invention can be usefully applied in a field where protein with a high concentration is required to be analyzed, for example, an analysis of protein using a sample analyzing tool in a clinical examination.

## Claims

1. A method for analyzing protein in a sample, in which the sample is alkaline, comprising:
adding a divalent copper salt and a salt containing a chaotropic ion to the sample;
allowing a Biuret reaction to proceed; and
analyzing the result optically

2. The method according to Claim 1, wherein, as the divalent copper salt and the salt containing the chaotropic ion, a divalent copper salt of a chaotropic ion is used.

3. The method according to Claim 1, wherein the chaotropic ion is at least one selected from the group consisting of a SCN ion, an I ion, a NO₃ ion, a ClO₄ ion, a Br ion and a Cl ion.

4. The method according to Claim 2, wherein the divalent copper salt of the chaotropic ion is at least one selected from the group consisting of Cu(NO₃)₂, Cu(ClO₄)₂, CuBr₂ and CuCl₂.

5. The method according to Claim 1, wherein an amount of the chaotropic ion to be added ranges from 0.5 mM to 100 mM.

6. The method according to Claim 1, wherein an amount of the chaotropic ion to be added ranges from 100 mM to 2000 mM.

7. The method according to Claim 1, wherein an amount of the divalent copper salt to be added ranges from 0.5 mM to 100 mM.

8. The method according to Claim 1, wherein an amount of the divalent copper salt to be added ranges from 100 mM to 2000 mM.

9. The method according to Claim 2, wherein an amount of the divalent copper salt of the chaotropic ion to be added ranges from 0.5 mM to 100 mM.

10. The method according to Claim 2, wherein an amount of the divalent copper salt of the chaotropic ion to be added ranges from 100 mM to 2000 mM.

11. The method according to Claim 1, wherein a pH of the sample being alkaline is 9 or more.

12. The method according to Claim 1, wherein a concentration of the protein in the sample ranges from 0 g/100mL to 15 g/100mL.

13. The method according to Claim 1, wherein the optical analysis is an analysis of a degree of coloring of the sample.

14. The method according to Claim 13, wherein the analysis of the degree of the coloring of the sample is performed by a visual evaluation, or by spectrophotometry or reflectometry using equipment.

15. The method according to Claim 1, wherein the optical analysis is a qualitative analysis or a quantitative analysis.

16. An analytical reagent for protein used in the method for analyzing protein according to Claim 1, comprising a divalent copper salt and a salt containing a chaotropic ion.

17. An analytical reagent for protein that is used in the method for analyzing protein according to Claim 2, comprising a divalent copper salt of a chaotropic ion.

18. The reagent according to Claim 16, wherein the chaotropic ion is at least one selected from the group consisting of a SCN ion, an I ion, a NO₃ ion, a ClO₄ ion, a Br ion and a Cl ion.

19. The reagent according to Claim 17, wherein the divalent copper salt of the chaotropic ion is at least one selected from the group consisting of Cu(NO₃)₂, Cu(ClO₄)₂, CuBr₂ and CuCl₂.

20. A sample analyzing tool, used in the method for analyzing protein according to Claim 1, comprising a carrier and a reagent,
wherein the reagent comprises a divalent copper salt and a salt containing a chaotropic ion.

21. A sample analyzing tool, used in the method for analyzing protein according to Claim 2, comprising a carrier and a reagent,
wherein the reagent comprises a divalent copper salt of a chaotropic ion.

22. The sample analyzing tool according to Claim 20, wherein the chaotropic ion is at least one selected from the group consisting of a SCN ion, an I ion, a NO₃ ion, a ClO₄ ion, a Br ion and a Cl ion.

23. The sample analyzing tool according to Claim 21, wherein the divalent copper salt of the chaotropic ion is at least one selected from the group consisting of Cu(NO₃)₂, Cu(ClO₄)₂, CuBr₂ and CuCl₂.
